# EUROPEAN PATENT SPECIFICATION

(11) **EP 1 178 841 B1**
(45) Date of publication and mention of the grant of the patent: **07.07.2004**
(21) Application number: 00932197.7
(22) Date of filing: 09.05.2000
(51) Int. Cl.: A61L 2/26

(54) **METHOD FOR CIRCUIT PROTECTION DURING RADIATION STERILIZATION**
VERFAHREN ZUR SCHUTZSCHALTUNG WÄHREND EINER BESTRAHLUNGSSTERILISATION
PROCEDE DE PROTECTION DE CIRCUIT DURANT UNE STERILISATION PAR RAYONNEMENT

(30) Priority: 21.05.1999 US 135279 P; 24.04.2000 US 556227
(43) Date of publication of application: 13.02.2002
(73) Proprietor: Medtronic MiniMed, Inc., Northridge, CA 91325-1219 (US)
(72) Inventor: VAN ANTWERP, William, P., Valencia, CA 91355 (US); KARRE, Sheana, Lincoln, NE 68512 (US); PROKOP, Adrian, Woodinville, WA 98072 (US); STINSON, Sara, Akiko, Alameda, CA 94502 (US); FONG, Jason, Pleasant Hill, CA 94523 (US); ROSENBERG, James, J., Monrovia, CA 91016 (US)
(74) Representative: Allman, Peter John
(86) International application number: PCT/US2000/012593
(87) International publication number: WO 2000/071173

(56) References cited:
- WO-A-98/34451
- GB-A- 1 529 968
- US-A- 5 397 735
- US-A- 5 496 302

## Description

### FIELD OF THE INVENTION

This invention relates to medical devices which contain sensitive semiconductor circuit elements and which protect the sensitive semiconductor circuit elements from high energy radiation sterilization.

### BACKGROUND OF THE INVENTION

Over the years, sterilization of medical devices has become more important and difficult. At one time it was possible to sterilize most medical devices at the site of use, since the devices were relatively simple, such as reusable syringes, scalpels, scissors or the like. In addition, the medical devices were often used in a doctor's office, hospital, or the like, that included sophisticated sterilization equipment. Thus, the medical devices could be shipped for use without sterilization.

However, medical devices have changed considerably over the years. For instance, the devices are becoming more complicated and use many different materials, such that autoclaving, chemical sterilization, or the like, is no longer possible, since the processes would likely destroy the materials of the medical device or fail to reach areas not readily accessible after construction of the medical device. In addition, many medical devices are now being used in the home by patients, and these patients generally do not have the equipment necessary to perform the required sterilization. Thus, on-site sterilization has become difficult or impossible to perform at the site of use.

To overcome this drawback, most devices are now sterilized at the facility of manufacture, prior to shipment. Sterilization can be facilitated by sterilization of each component prior to assembly in a sterilized environment. However, additional sterilization is often required. Heat and chemical sterilization cannot always be used due to possible destruction of the various materials. To overcome issues of heat and chemical sterilization, radiation (including x-ray and electron beam) sterilization was developed to provide sterilization of a medical device once it was in its packaging. Thus, after sterilization, the device is shipped and stored in a sterilized environment until the medical device is to be used.

Although radiation sterilization has solved many sterilization issues, it is difficult to sterilize complicated medical devices containing electronic circuits, since the radiation has the capability to damage or destroy semiconductor circuit elements. One approach is to remove the circuits prior to sterilization, but this still raises issues of sterilization when the medical device is reassembled.

### SUMMARY OF THE DISCLOSURE

According to the present invention defined in appended claim 1 there is provided a method for sterilizing a medical device using high energy radiation selected from one of electron beam sterilization, gamma ray sterilization, x-ray sterilization or proton beam sterilization, wherein the medical device is packaged in a metallic protective housing that is hermetically coupled to a support substrate that supports sensitive semiconductor circuit elements, the high energy radiation is stopped by the metallic protective housing and by-products are formed by the stopping process, and the by-products are absorbed by an energy absorbing material contained within the area sealed by the protective housing.

In further embodiments, the medical device includes a protective conductor that is coupled to the support substrate on a side which is opposite the protective housing to prevent high energy from entering the opposite side of the support substrate. Preferably the support substrate is a circuit board. Also, the high energy sterilization is electron beam sterilization and the byproducts are x-rays.

In particular embodiments, the energy absorbing material is an epoxy containing metal. Also, the protective housing and protective conductor are formed from a metal or other electrical conductor. For instance, the metal is selected from the group consisting essentially of titanium and aluminum.

In particular embodiments, the predetermined exposure level is above 0.5 Mrad, but is more preferably above 2.0 Mrad. Alternatively, the predetermined exposure level is less than or equal to 5.0 Mrad.

Other features and advantages of the invention will become apparent from the following detailed description, taken in conjunction with the accompanying drawings which illustrate, by way of example, various features of embodiments of the invention.

### BRIEF DESCRIPTION OF THE DRAWINGS

A detailed description of embodiments of the invention will be made with reference to the accompanying drawings, wherein like numerals designate corresponding parts in the several figures.
Fig. 1 is a perspective view of a circuit board that uses a circuit protection device useful for the present invention.
Fig. 2 is a partial cross-sectional diagram of the circuit protection device as shown along the line 2-2 in Fig. 1.
Fig. 3 is a cross-sectional diagram that shows a circuit board with a circuit protection device, as shown in Figs. 1 and 2, that is contained in a medical device and is exposed to E-beam sterilization.

### DETAILED DESCRIPTION OF THE PREFERRED EMBODIMENTS

As shown in the drawings for purposes of illustration, the invention is embodied in a method for use during E-beam sterilization to permit sterilization of a medical device containing semiconductor circuit elements. In preferred embodiments of the present invention, the circuit protection device forms a permanent part of a circuit board, which permits sterilization of the medical device and circuit board after final assembly. However, it will be recognized that further embodiments of the invention may be used to protect circuit elements that are sterilized prior to final assembly in a medical device. The circuit protection device is primarily adapted for use in medical device for external use. However, alternative embodiments may be used in medical devices to be placed internally within the patient or for both internal and external use. Preferred embodiments are for use with medication infusion devices. However, alternative embodiments may be used with other medical devices containing E-beam sensitive components. Preferred embodiments of the present invention are directed to protecting circuits during E-beam sterilization. However, other embodiments, may protect circuits during other sterilization techniques that utilize other energy sources, such as gamma ray, x-ray, proton beam, or the like.

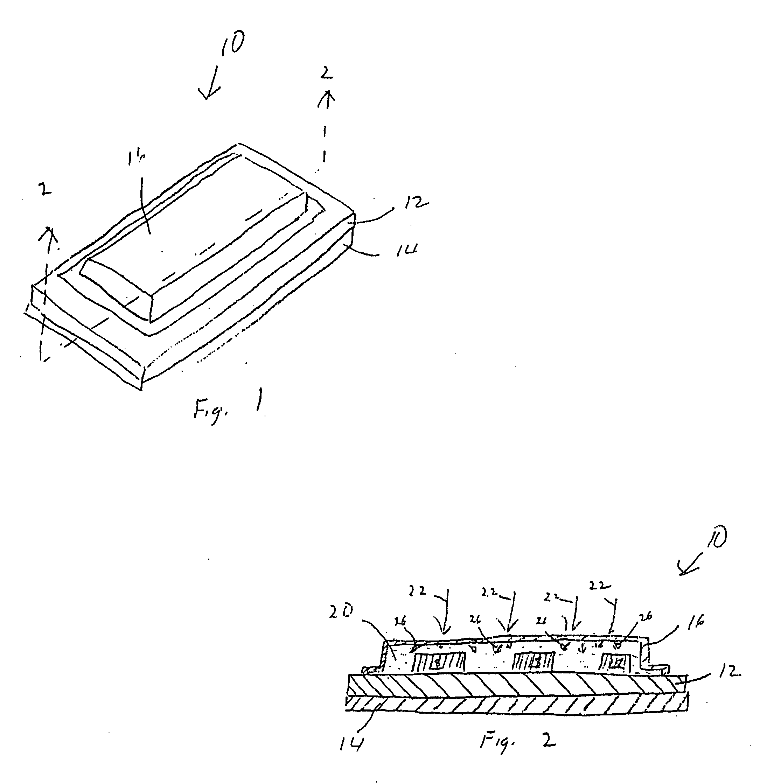

As shown in Figs. 1-3, the circuit protection device 10 useful for the present invention includes a circuit board 12 (or support substrate), a protective conductor 14 and a protective housing 16 to cover E-beam sensitive components 18 mounted on the circuit board 12. In preferred embodiments, the E-beam sensitive components are potted (or surrounded) with an energy absorbing substance 20 that further protects the E-beam sensitive components 18 mounted on the circuit board 12.

Preferred embodiments of the protective housing 16 are sealed the circuit board 12 to provide a hermetic seal. Thus, once the exterior of the protective housing 16 and the circuit board are sterilized, the entire component assembly is sterilized, since no contaminates inside the sealed environment around the E-beam sensitive components 18 can escape to effect the sterilized device. In preferred embodiments, the protective housing 16 is attached to the circuit board 12 using an adhesive, such as RTV, silicone based adhesives, epoxies, or the like. Also, if the energy absorbing material 20 is used and has adhesive properties, it may be used to secure the protective housing 16 and form the hermetic seal. In other embodiments, the protective housing 16 may be welded to the circuit board 12, or include a plastic liner that permits sonic welding of the protective housing 16 to the circuit board 14. In further embodiments, the protective housing 16 may be used with a gasket (not shown) and snapped in place or otherwise secured to the circuit board 12 to form a hermetic seal.

Preferably, the protective housing 16 is formed of an electron stopping light metal such as aluminum or titanium. In alternative embodiments, other materials may be used that stop electrons or radiation, such as silver, gold, lead, tantalum, or the like or other electrically conductive materials. Preferably, the protective housing 16 is formed from a single sheet of stamped metal to facilitate assembly and to reduce cost. However, alternative embodiments may use other structures, such as cast metals, laminates, or the like. In addition, the protective conductor 14 should be formed of a similar electron stopping material to prevent electrons from passing through the back of the circuit board 12 to the E-beam sensitive components 18.

Preferred embodiments of the protective housing 16 and protective conductor 14 have a thickness that is sufficient to withstand electron beam sterilization and stop the electrons with a preferred single dose of 2.0. Mrads (or 20 kGy). However, in alternative embodiments, smaller dose levels may be used if sufficient sterilization may be achieved at the lower dose, such as for example 0.5 Mrads (5 kGy). Larger doses may also be used, if the protective housing 16 and protective conductor 14 (as well as other medical device components - not shown) are selected and assembled to withstand doses up to 5.0 Mrads (50 kGy). The circuit protection device 10 materials are carefully selected with regard to protective housing materials, circuit board materials, electrodes, any membranes, chemistry, lubricants, and the packaging materials, and manufacturing tolerances to assure the ability to withstand electron beam sterilization and the continued proper operation of the E-beam sensitive components 18 after sterilization.

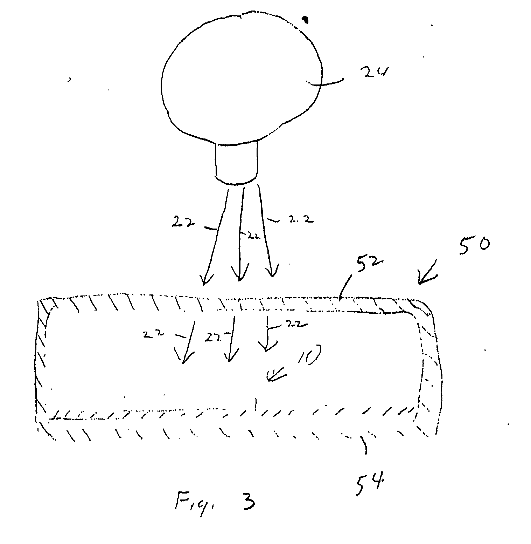

In operation, as shown in Figs. 2 and 3, the protective housing 16 stops the electrons in the E-beam 22 from an E-beam source 24 from reaching and impinging on the E-beam sensitive components 18 after passing through the walls 52 of a medical device 50. In addition, the protective conductor 14 prevents electrons in the E-beam 22 from reflecting off of a back wall 54 in the medical device 50 and damaging the E-beam sensitive components 18 by passing through the back of the circuit board 12. Thus, the structure of the of circuit protection device 10 formed by the circuit board 12, the protective conductor 14 and the protective housing forms a small Faraday cage to protect the enclosed E-beam sensitive components 18 from damaging electrons during the sterilization procedure.

Light metals, as discussed above, are particularly well adapted to stopping electrons in E-beams 22. However, a drawback to the use of light metals is that they often produce x-rays 26 (see Fig. 2) as a byproduct from the stopping of the electrons in the E-beams 22. Therefore, to protect the E-beam sensitive components 18, the interior area under the protective housing 16 is filled with an energy absorbing material 20 that is particularly well suited for absorbing x-rays, or electron stopping energy byproducts. In preferred embodiments, the energy absorbing material is an epoxy that contains a metal to stop and absorb the x-rays. Any such compound should be selected to avoid interfering with any electrical operation of the E-beam sensitive components 18. In alternative embodiments, the energy absorbing material is a liner (not shown) in a laminate structure forming the protective conductor 14 and protective housing 16, such as lead, or the like, that stops and absorbs x-rays. In further alternatives, other energy absorbing materials may be used.

Preferred embodiments of the E-beam sensitive components 18 are semiconductor devices, such as microprocessors, RAMs, ROMS, flash memory, or the like. However, alternative embodiments, may include other E-beam sensitive components, such as temperature sensors, antennas, power sources, batteries, or the like. If the E-beam sensitive components 18 generate heat (or need to conduct heat), then it is preferred that any energy absorbing material 20 act as a conductor to maintain temperature equilibrium within the medical device 50.

## Claims

1. A method for sterilizing a medical device using high energy radiation selected from one of electron beam sterilization, gamma ray sterilization, x-ray sterilization or proton beam sterilization, wherein the medical device is packaged in a metallic protective housing (10) that is hermetically coupled to a support substrate (12) that supports sensitive semiconductor circuit elements (18), the high energy radiation is stopped by the metallic protective housing (10) and by-products are formed by the stopping process, and the by-products are absorbed by an energy absorbing material (20) contained within the area sealed by the protective housing (10).

2. The method according to claim 1 wherein a protective conductor (14) is coupled to the support substrate (12) on a side opposite the protective housing (10) to prevent high energy radiation from entering the side of the support substrate (12).

3. The method according to claim 1 or 2 wherein the high energy radiation sterilization is electron beam sterilization and the by products are x-rays.

4. The method according to any preceding claim wherein the energy absorbing material is an epoxy containing metal.

5. The method according to any preceding claim wherein the predetermined exposure level is above 0.5 Mrad.

6. The method according to claim 5 wherein the predetermined exposure level is above 2.0Mrad.

7. The method according to any preceding claim wherein the predetermined exposure level is less than or equal to 5.0Mrad.

## Patentansprüche

1. Verfahren zur Sterilisation eines medizinischen Gerätes bei Anwendung einer energiereichen Strahlung, ausgewählt unter einem von Elektronenstrahlsterilisation, Gammastrahlensterilisation, Röntgenstrahlensterilisation oder Protonenstrahlensterilisation, bei dem das medizinische Gerät in ein Metallschutzgehäuse (10) verpackt wird, das hermetisch mit einem Trägersubstrat (12) gekoppelt ist, das empfindliche Halbleiterschaltelemente (18) trägt, die energiereiche Strahlung durch das Metallschutzgehäuse (10) gestoppt wird und Nebenprodukte durch den Stoppvorgang gebildet werden und die Nebenprodukte durch ein energieabsorbierendes Material (20) absorbiert werden, das innerhalb der Fläche enthalten ist, die durch das Schutzgehäuse (10) abgedichtet wird.

2. Verfahren nach Anspruch 1, bei dem ein Schutzleiter (14) mit dem Trägersubstrat (12) auf einer Seite gekoppelt ist, die dem Schutzgehäuse (10) entgegengesetzt ist, um zu verhindern, daß energiereiche Strahlung in die Seite des Trägersubstrates (12) eintritt.

3. Verfahren nach Anspruch 1 oder 2, bei dem die Sterilisation mittels energiereicher Strahlung die Elektronenstrahlsterilisation ist und die Nebenprodukte Röntgenstrahlen sind.

4. Verfahren nach einem der vorhergehenden Ansprüche, bei dem das energieabsorbierende Material ein epoxidhaltiges Metall ist.

5. Verfahren nach einem der vorhergehenden Ansprüche, bei dem das vorgegebene Bestrahlungsniveau über 0,5 Mrad beträgt.

6. Verfahren nach Anspruch 5, bei dem das vorgegebene Bestrahlungsniveau über 2,0 Mrad beträgt.

7. Verfahren nach einem der vorhergehenden Ansprüche, bei dem das vorgegebene Bestrahlungsniveau kleiner als oder gleich 5,0 Mrad ist.

## Revendications

1. Procédé de stérilisation d'un dispositif médical utilisant un rayonnement d'énergie élevée sélectionné parmi la stérilisation par faisceau électronique, la stérilisation par rayons gamma, la stérilisation par rayons X ou la stérilisation par faisceau protonique, dans lequel le dispositif médical est emballé dans un boîtier de protection métallique (10) couplé hermétiquement à un substrat support (12) qui supporte des éléments de circuit semi-conducteurs sensibles (18), le rayonnement d'énergie élevée est arrêté par le boîtier de protection métallique (10) et des produits dérivés sont formés par le procédé d'arrêt, et les produits dérivés sont absorbés par un matériau absorbant l'énergie (20) contenu dans la zone hermétiquement fermée par le boîtier de protection (10).

2. Procédé selon la revendication 1, dans lequel un conducteur de protection (14) est couplé au substrat support (12) d'un côté opposé au boîtier de protection (10) pour empêcher le rayonnement d'énergie élevée de pénétrer dans le côté du substrat support (12).

3. Procédé selon la revendication 1 ou 2, dans lequel la stérilisation par rayonnement d'énergie élevée est une stérilisation par faisceau électronique et les produits dérivés sont des rayons X.

4. Procédé selon l'une quelconque des revendications précédentes, dans lequel le matériau absorbant l'énergie est un métal contenant de l'époxy.

5. Procédé selon l'une quelconque des revendications précédentes, dans lequel le niveau d'exposition prédéterminé est supérieur à 0,5 Mrad.

6. Procédé selon la revendication 5, dans lequel le niveau d'exposition prédéterminé est supérieur à 2,0 Mrad.

7. Procédé selon l'une quelconque des revendications précédentes, dans lequel le niveau d'exposition prédéterminé est inférieur ou égal à 5,0 Mrad.
